# FASCICULE DE BREVET EUROPEEN

(11) **EP 3 233 820 B1**
(45) Date de publication et mention de la délivrance du brevet: **15.05.2019**
(21) Numéro de dépôt: 15828740.9
(22) Date de dépôt: 14.12.2015
(51) Int. Cl.: C07D 317/12, A61K 8/49, A61Q 13/00, C07D 319/06, C07D 321/06

(54) **NOUVEAUX ACÉTALS DE LA 1-(3,3-DIMÉTHYLCYCLOHEX-1-ÈNYL) ÉTHANONE, LEUR PROCÉDÉ DE PRÉPARATION AINSI QUE LEUR UTILISATION EN PARFUMERIE**
NEUARTIGE ACETALE VON 1-(3,3-DIMETHYLCYCLOHEX-1-ENYL)-ETHANON, VERFAHREN ZUR HERSTELLUNG DAVON UND VERWENDUNG DAVON IN DER PARFÜMERIE
NOVEL ACETALS OF 1-(3,3-DIMETHYLCYCLOHEX-1-ENYL) ETHANONE, METHOD FOR THE PRODUCTION THEREOF AND USE OF SAME IN PERFUMERY

(30) Priorité: 18.12.2014 FR 1462834
(43) Date de publication de la demande: 25.10.2017
(73) Titulaire: V. Mane Fils, 06620 Le Bar sur Loup (FR)
(72) Inventeur: MURATORE, Agnès, 06740 Châteauneuf (FR); MAHAIM, Cyril, 1052 Le Mont-sur-Lausanne (CH)
(74) Mandataire: Murgitroyd & Company
(86) Numéro de dépôt international: PCT/FR2015/053495
(87) Numéro de publication internationale: WO 2016/097569

(56) Documents cités:
- FR-A1- 2 259 091
- US-B1- 6 348 618

## Description

La présente invention a pour objet de nouveaux acétals de la 1-(3,3-diméthylcyclohex-1-ènyl)éthanone possédant des notes ambrées ou animales, leur procédé de préparation, ainsi que leurs utilisations dans l'industrie de la chimie, et en particulier en parfumerie, cosmétique, et dans l'industrie des détergents, lesdits composés présentant une fragrance et une rémanence particulières.

L'industrie des parfums est toujours à la recherche de composés organoleptiques nouveaux, présentant un pouvoir olfactif intense, tout en ayant des coûts de production les plus limités possibles. Plus particulièrement, des composés exhibant des notes ambrées ou animales sont rares et difficiles à obtenir. Mais plus encore, obtenir des composés ayant des notes ambrées ou animales tout en ne présentant pas de note boisé, le tout à un faible coût de revient, devient de moins en moins aisé.

Parmi les molécules organoleptiques d'intérêt en parfumerie, les composés dotés de notes ambrées et/ou animales sont rares. On compte, parmi les plus utilisés l'Ambrinol® (Firmenich, Suisse) ou 2,5,5-triméthyl-1,2,3,4,4a,5,6,7-octahydronaphthalèn-2-ol (DE 2733928) ou le Grisalva® (IFF, USA) ou 3a-éthyl-6,6,9a-triméthyldodecahydronaphtho[2,1-c]furanne (US 7468447), tous deux représentés ci-dessous :

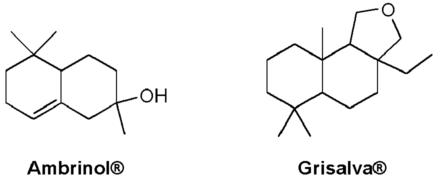

Mais l'inconvénient principal de beaucoup de composés dotés d'une note ambrée et/ou animale, tient en la complexité de leurs procédés de synthèse qui en font des matières premières très onéreuses et difficiles à obtenir à rendement élevé. Par exemple, l'Ambrinol® peut être préparé en deux étapes à partir de la β-ionone (US 4163866) ; cependant la première étape de thermolyse effectuée entre 300 et 500 °C ne conduit à un intermédiaire déhydroambrinol (précurseur de l'ambrinol) qu'à hauteur de 27%. De plus, la deuxième étape d'hydrogénation fournit un mélange d'isomères α- et β-ambrinol alors que seul l'isomère α-ambrinol est désiré. De même, le procédé de synthèse du Grisalva® comporte pas moins de neuf étapes à partir du 1-(2,2,6,-triméthyl-cyclohexyl)-pentan-3-one (US 7468447).

Aussi, afin de faire face aux besoins constants de l'industrie des parfums et arômes, la Demanderesse a identifié de nouveaux dérivés acétals de la 1-(3,3-diméthylcyclohex-1-ènyl)éthanone, ces composés présentant une note ambrée ou animale très puissante et diffusive, ainsi qu'une rémanence remarquable.
Ces dérivés acétals de la 1-(3,3-diméthylcyclohex-1-ènyl)éthanone répondent à la formule générale I suivante : 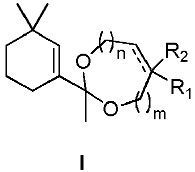 dans laquelle :
- m et n représentent un nombre de carbone (groupe méthylène) et sont chacun indépendamment 0 ou 1;
- R₁ représente un atome d'hydrogène ou un groupe alkyle en C1-C2 saturé;
- la liaison carbone-carbone en pointillé est présente ou absente, et
   lorsque ladite liaison est absente, R₂ est présent et représente un atome d'hydrogène ou un groupe alkyle en C1-C2 saturé,
   lorsque ladite liaison est présente, R₂ est absent;
   ledit composé étant sous forme d'un stéréoisomère, d'un mélange de stéréoisomères, ou d'un mélange racémique.

Selon un mode de réalisation préféré, m et n représentent un nombre de groupement -CH₂- et sont chacun indépendamment 0 ou 1.

La présente invention se rapporte également à une composition comprenant au moins un composé de formule générale I.

En outre, un troisième objet de la présente invention se rapporte à un procédé de préparation d'un composé de formule générale I, ledit procédé étant simple, efficace en terme de rendement, comportant un faible nombre d'étapes et donc peu onéreux.

Enfin, un dernier objet de la présente invention concerne l'utilisation d'au moins un composé de formule générale I en tant qu'agent fragrant.

A la connaissance de la Demanderesse, aucun des composés répondant à la formule générale I n'a été identifié auparavant.
Certains dérivés acétals de la 1-(3,3-diméthylcyclohexyl)éthanone ont été décrits dans la demande de brevet EP 0472966, notamment le 2-(3,3-diméthylcyclohexyl)-2,4-diméthyl-1,3-dioxolane et le 2-(3,3-diméthylcyclohexyl)-2,4,4-triméthyl-1,3-dioxolane) Cependant, lesdits dérivés y sont décrits simplement en tant qu'intermédiaires de synthèse et aucune description olfactive ne leur est associée.

Aussi, l'art antérieur décrit-il d'autres composés acétals représentés ci-dessous, comme le Karanal® (Givaudan, Suisse) ou 5-sec-butyl-2-(2,4-diméthylcyclohex-3-ènyl)-5-méthyl-1,3-dioxane (EP 0276998), l'Ysamber K® (Dragoco, Japon) ou spiro[1,3-dioxolane-2,8'(5'h)-[2h-2,4a]méthanonaphthalène] (EP 543470), ou encore l'Amberketal® (Firmenich, Suisse) ou dodecahydro-3,8,8,11a-tetraméthyl-5h-3,5a-epoxynaphth[2,1-c]oxepin (US 3144465).

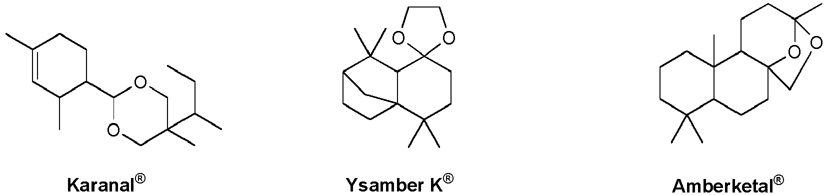

Cependant, ces composés possèdent d'une part, une structure chimique bien différente de celle des composés de la présente invention et, d'autre part, des notes ambrées associées à des notes boisées. De manière similaire, les cétones de formule I objet du brevet FR-B-2259091, telles que le composé 1-(3,3-diméthyl-cyclohex-1-ène-1-yl)-1,1-éthylènedioxy-pent-4-ène), et certains de leurs acétals, ont également une structure chimique bien différente de celle des composés de la présente invention et présentent, là encore, une note boisée. A contrario, les composés selon la présente invention ont l'avantage de présenter des notes animales ou ambrées dépourvues d'un aspect boisé.

Ainsi, l'invention a pour objet un composé de formule générale I suivante : 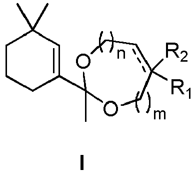 dans laquelle :
- m et n représentent un nombre de carbone (groupe méthylène) et sont chacun indépendamment 0 ou 1;
- R₁ représente un atome d'hydrogène ou un groupe alkyle en C1-C2 saturé;
- la liaison carbone-carbone en pointillé est présente ou absente, et
   lorsque ladite liaison est absente, R₂ est présent et représente un atome d'hydrogène ou un groupe alkyle en C1-C2 saturé,
   lorsque ladite liaison est présente, R₂ est absent;
   ledit composé étant sous forme d'un stéréoisomère, d'un mélange de stéréoisomères, ou d'un mélange racémique.

Tel qu'indiqué précédemment, selon un mode de réalisation préféré, m et n représentent un nombre de groupement -CH₂- et sont chacun indépendamment 0 ou 1.

Au sens de la présente invention, le terme « alkyle en C₁-C₂ » désigne tout radical monovalent dérivé d'une chaîne carbonée saturée, linéaire, contenant 1 ou 2 atomes de carbone, c'est à dire un groupement méthyle ou éthyle.

Selon un premier mode de réalisation, la liaison carbone-carbone en pointillé est absente. Préférentiellement, n est égal à zéro et m est égal à 1.

Selon un autre mode de réalisation préféré, n et m sont égaux à zéro.

Dans un dernier mode de réalisation, les groupements R₁ et R₂ représentent un groupe alkyle en C₁-C₂ saturé.

Encore plus préférentiellement, le composé selon la présente invention est choisi parmi le 2-(3,3-diméthylcyclohex-1-ènyl)-2-méthyl-1,3-dioxolane, le 2-(3,3-diméthylcyclohex-1-ènyl)-2,4-diméthyl-1,3-dioxolane, le 2-(3,3-diméthylcyclohex-1-ènyl)-2-méthyl-1,3-dioxane, le 2-(3,3-diméthylcyclohex-1-ènyl)-2,5-diméthyl-1,3-dioxane, le 2-(3,3-diméthylcyclohex-1-ènyl)-2,5,5-triméthyl-1,3-dioxane, le 2-(3,3-diméthylcyclohex-1-ènyl)-5,5-diéthyl-2-méthyl-1,3-dioxane et le 2-(3,3-diméthylcyclohex-1-ènyl)-2-méthyl-4,7-dihydro-1,3-dioxepine.

La présence de centres d'asymétrie dans la structure des composés de formule I selon l'invention provoque l'existence, pour chacun d'entre eux, de plusieurs formes énantiomériques et/ou diastéréomériques. L'invention couvre également les composés représentés par la formule générale I sous forme de mélanges d'énantiomères et/ou de diastéréomériques, en proportions variables, en particulier les mélanges racémiques. L'invention comprend également les composés de formule I sous forme d'un seul énantiomère et/ou diastéréomère. Des mélanges d'énantiomères/ diastéréomères ou des formes pures peuvent être obtenus par synthèse à partir de produits de départ optiquement enrichis ou optiquement purs, ou au moyen de méthodes de séparation par cristallisation ou chromatographie.

Un second objet de la présente invention se rapporte à une composition comprenant au moins un composé de formule générale I tel que définie précédemment. De par l'odeur plaisante qu'ils dégagent, les composés de l'invention trouvent nombre d'applications en parfumerie. Le terme "parfumerie" est ici utilisé dans son sens général ; il désigne non seulement la parfumerie traditionnelle (alcoolique ou non), mais également les autres domaines dans lesquels l'odeur des produits est importante. Il peut être ainsi fait référence à des compositions de parfumerie au sens habituel et traditionnel (telles que bases et concentrés parfumant, parfums, eaux de Cologne, eaux de toilette, désodorisants d'intérieur, parfums d'ambiance, bougie parfumées et produits similaires), à des compositions topiques notamment cosmétiques (telles que crèmes pour le visage et/ou le corps, poudres de talc, huiles pour cheveux, shampooings, lotions capillaires, sels et huiles de bain, gels de douche et/ou de bain, savons de toilette, antiperspirants et déodorants corporels, lotions et crèmes de rasage, savons, dentifrices, bains de bouche, pommades, et produits similaires), ainsi qu'à des produits d'entretien, notamment ménagers (tels que détergents, lessives, assouplissants, désodorisants d'intérieur, parfums d'ambiance et produits similaires).

L'invention s'étend ainsi à une composition de parfum comprenant au moins un composé de l'invention. Il peut notamment s'agir d'une composition de la parfumerie traditionnelle, d'une composition cosmétique, de produit d'entretien, ou encore d'une "composition dite intermédiaire", destinée à être utilisée pour la préparation de compositions ou de produits finis (notamment parfums, produits cosmétiques, produits d'entretien).

Une telle composition parfumée est généralement préparée à partir d'un produit de base, dans lequel le ou les composés de l'invention se trouvent être incorporés. Le produit de base sera aisément déterminé par l'homme du métier en fonction de la composition envisagée et donc de l'utilisation envisagée. La composition de ces produits de base et la nature de leurs composants habituels, tels que solvant(s) et/ou adjuvant(s), sont bien connues de l'homme du métier.

Les composés entrant dans ces compositions parfumées, notamment les composés de l'invention, peuvent être incorporés dans ou sur un matériau support inerte. Les matériaux supports pouvant êtres employés sont nombreux et variés, par exemple des solvants polaires, des huiles, des graisses, des solides finement divisés, des cyclodextrines, des maltodextrines, des gommes, des résines et n'importe quel autre matériau support connu pour de telles compositions (par exemple, savons, bougies, pommades, textiles, lingettes, gels parfumés...).

Préférentiellement, une composition selon la présente invention comprend au moins un composé choisi parmi le 2-(3,3-diméthylcyclohex-1-ènyl)-2-méthyl-1,3-dioxolane, le 2-(3,3-diméthylcyclohex-1-ènyl)-2,4-diméthyl-1,3-dioxolane, le 2-(3,3-diméthylcyclohex-1-ènyl)-2-méthyl-1,3-dioxane, le 2-(3,3-diméthylcyclohex-1-ènyl)-2,5-diméthyl-1,3-dioxane, le 2-(3,3-diméthylcyclohex-1-ènyl)-2,5,5-triméthyl-1,3-dioxane, le 2-(3,3-diméthylcyclohex-1-ènyl)-5,5-diéthyl-2-méthyl-1,3-dioxane et le 2-(3,3-diméthylcyclohex-1-ènyl)-2-méthyl-4,7-dihydro-1,3-dioxepine.

La quantité efficace des composés de l'invention à incorporer dans ces compositions est fonction de la nature desdites compositions, de l'effet odorant souhaité et de la nature des autres composés odorants éventuellement présents. Elle est aisément déterminée par l'homme du métier, et peut varier dans une plage très étendue, de 0,1 à 99%, en particulier 0,1 à 50%, notamment 0,1 à 30% en poids par rapport au poids total de la composition. Les pourcentages précédents sont exprimés en poids total de la composition.

Selon un dernier mode de réalisation particulier, la composition est caractérisée en ce qu'elle comprend en outre au moins un autre agent fragrant/substance odorante. Les agents fragrants/substances odorantes pouvant être utilisés en combinaison avec les composés de la présente invention peuvent être des produits naturels tels que des extraits, des huiles essentielles, des absolues, des résinoïdes, des résines, des concrètes etc., mais aussi des produits de synthèse tels que des hydrocarbures, alcools, aldéhydes, cétones, éthers, acides, esters, acétals, nitriles etc., notamment des composés saturés ou insaturés, aliphatiques, hétérocycliques ou carbonecycliques. De tels agents fragrants/substances odorantes sont mentionnés par exemple, dans S. Arctander, « Perfume and Flavor Chemicals » (Montclair, N.J., 1969), ou encore dans « Common Fragrance and Flavor Materials », Wiley-VCH, Weinheim, 2006. Enfin, plusieurs composés de la présente invention peuvent également être utilisés en combinaison dans une même composition.

Un troisième objet de la présente invention se rapporte à un procédé de synthèse des composés de formule I tel que définis précédemment.

Ledit procédé est avantageux en ce sens qu'il permet l'utilisation de matières premières disponibles en grande quantité, la mise en oeuvre de conditions réactionnelles douces et facilement adaptables aux chaînes de production existantes, ainsi que l'évincement de réactifs susceptibles de nuire à la santé ou à l'environnement en vue d'une application industrielle.

Les composés de la présente invention sont obtenus grâce à un procédé comprenant les étapes suivantes de :
a) cyclisation/réarrangement du dehydrolinalol par un acide, en 1-(3,3-diméthylcyclohex-1-ènyl)éthanone et ;
b) acétalisation de la 1-(3,3-diméthylcyclohex-1-ènyl)éthanone par un diol pour obtenir un composé de formule I.

Le dehydrolinalol (DE 1643710) est une matière première facilement accessible et peu chère, permettant ainsi une fabrication simple et compatible avec les exigences de l'industrie. Préférentiellement, la réaction de cyclisation/réarrangement du déhydrolinalol se fait par ajout d'un acide choisi parmi l'acide phosphorique ou encore l'acide méthanesulfonique, et conduit à la 1-(3,3-diméthylcyclohex-1-ènyl)éthanone.
L'étape b) du procédé selon l'invention est réalisée par addition d'un diol au composé obtenu lors de l'étape a), à savoir la 1-(3,3-diméthylcyclohex-1-ènyl)éthanone. Préférentiellement, le diol est choisi parmi l'éthylène glycol, le néopentyl glycol, le 1,2-propanediol, le 2,2-diéthyl-1,3-propanediol, le 2-méthyl-1,3-propanediol, le 1,3-propanediol, ou encore le *cis*-2-butène-1,4-diol. Cette étape d'acétalisation est conduite en présence de triéthyle orthoformiate et d'une faible quantité d'un acide qui joue le rôle de catalyseur et présente l'avantage d'être régénéré (d'où une économie du coût du procédé). Par faible quantité, on entend une quantité égale à 1% de la masse de 1-(3,3-diméthylcyclohex-1-ènyl)éthanone introduite dans le milieu réactionnel. L'acide *para*-toluène sulfonique est par exemple un acide utilisable pour cette étape b) .

Avantageusement, il est possible de réaliser l'étape b) du procédé directement à partir du déhydroherbac. De manière consacrée, la dénomination « déhydroherbac » désigne un mélange des isomères 1-(3,3-diméthylcyclohex-1-ényl)éthanone et 1-(3,3-diméthylcylohex-6-ényl)éthanone (US4264467), la proportion relative entre ces 2 isomères important peu. Des formes pures de ces isomères peuvent être obtenues à partir du déhydroherbac grâce à des méthodes de séparation bien connues, comme la séparation par cristallisation et/ou chromatographie. Il est également possible de partir du mélange dehydroherbac ; la réaction dans ce cas aura lieu sur l'isomère 1-(3,3-diméthylcyclohex-1-ényl)éthanone uniquement, l'isomère 1-(3,3-diméthylcyclohex-6-ényl)éthanone ne réagissant que très faiblement.
Ainsi, le procédé selon la présente invention présente les avantages d'être réalisé sans solvant, mais également sans eau grâce à l'utilisation de triéthyle orthoformiate, d'être conduite à température ambiante en quelques heures, et enfin d'avoir un rendement compris entre 70% et 80%.

Enfin, l'invention a pour dernier objet l'utilisation d'au moins un composé de formule I selon l'invention comme agent ou composé fragrant, comme agent de masquage d'odeur ou comme agent de neutralisation d'odeur. Le terme «fragrant», est utilisé ici pour désigner tout composé organoleptique stimulant de façon plaisante l'odorat. Par le terme «agent de masquage» ou «masquant» on entend réduire ou éliminer la perception d'une mauvaise odeur générée par une ou plusieurs molécules entrant dans la composition d'un produit.

En outre, ledit composé peut être utilisé seul ou en combinaison avec au moins un autre agent fragrant/une autre substance odorante et/ou au moins un solvant et/ou au moins un adjuvant. Le ou les agents fragrants/odorants supplémentaires, solvants et adjuvants sont connus de l'homme du métier qui sera à même de choisir le ou les plus appropriés en fonction de l'effet recherché.

Un mode de réalisation particulier de l'invention réside en l'utilisation d'un composé de formule I pour modifier ou renforcer les propriétés organoleptiques d'une substance, d'une composition ou d'un article.

On entend par «propriétés organoleptiques» toute propriété susceptible de modifier, améliorer ou renforcer la perception organoleptique d'une substance, d'une composition, d'un article par un utilisateur. Ainsi, à titre d'exemple préférentiel, l'agent organoleptique selon l'invention peut consister en un agent parfumant susceptible de conférer, modifier, améliorer ou renforcer la perception olfactive d'une substance, d'une composition ou d'un article.

Les exemples suivants illustrent une manière particulière de préparer les composés de l'invention, ainsi que le profil olfactif de chacun des composés exemplifiés. Ces exemples ne sont donnés que dans un but d'illustration et ne doivent pas être compris comme limitant la portée générale de l'invention.

### Exemple 1 : Préparation du 2-(3,3-diméthylcyclohex-1-ènyl)-2-méthyl-1,3-dioxolane

Dans un ballon, on place un équivalent de dehydrolinalol dans du toluène. On porte cette solution à reflux. De l'acide phosphorique (10% de la masse de dehydrolinalol) est alors ajouté lentement par un goutte à goutte. Après 20 heures d'agitation à reflux, et une fois revenu à température ambiante, on verse le milieu réactionnel sur une solution aqueuse saturée de bicarbonate de sodium. Les phases sont séparées. La phase organique est lavée à l'eau jusqu'à neutre. Après séchage sur sulfate de magnésium, filtration et évaporation du solvant, le produit brut est distillé sous pression réduite : sa température d'ébullition est de 52 °C sous 53.3 Pascal.
La 1-(3,3-diméthylcyclohex-1-ènyl)éthanone ainsi obtenue est placée dans 4 équivalents d'éthylène glycol et 2 équivalents de triéthyle orthoformiate. A température ambiante, on ajoute de l'acide *para*-toluène sulfonique (1% de la masse de 1-(3,3-diméthylcyclohex-1-ènyl)éthanone introduite). Le milieu réactionnel est laissé sous agitation dans ces conditions pendant vingt heures, puis versé sur une solution aqueuse saturée en bicarbonate de sodium. Cette phase aqueuse est extraite deux fois au cyclohexane. Les phases organiques réunies sont lavées à l'eau jusqu'à neutre. Après séchage sur sulfate de magnésium, filtration et concentration, le produit brut est distillé sous pression réduite : sa température d'ébullition est de 46 °C sous 53.3 Pascal.

### Description olfactive : animal, ambré, aromatique, terpénique.

Le 2-(3,3-diméthylcyclohex-1-ènyl)-2-méthyl-1,3-dioxolane ainsi obtenu présente les caractéristiques spectrales suivantes :
¹H-NMR (200 MHz, CDCl₃) : δ (ppm) 0,94 (s, 6H), 1,34-1,37 (m, 2H), 1,41 (s, 3H), 1,57-1,61 (m, 2H), 1,91 (td, *J* = 6,2 Hz, 1,6 Hz, 2H), 3, 70-3, 85 (m, 2H), 3,85-3,95 (m, 2H), 5,56 (s, 1H) .
¹³C-NMR (50 MHz, CDCl₃) : δ (ppm) 19, 85, 23, 76, 23, 96, 29, 92, 31, 34, 36, 95, 64, 18, 109, 31, 132,60, 134,75.
MS [e/m (%)]: 196 (M+, 0,2), 181 (100), 109 (21), 87 (82), 73 (14), 43 (32).
IR (film, cm⁻¹) : 859m, 946w, 1043s, 1110w, 1187m, 1209m, 1371w, 2933m.

### Exemple 2 : Préparation du 2-(3,3-diméthylcyclohex-1-ènyl)-2,4-diméthyl-1,3-dioxolane

Le 2-(3,3-diméthylcyclohex-1-ènyl)-2,4-diméthyl-1,3-dioxolane est préparé selon le protocole décrit en exemple 1 en utilisant le 1,2-propanediol à la place de l'éthylène glycol. Le produit brut constitué de 2-(3,3-diméthylcyclohex-1-ènyl)-2,4-diméthyl-1,3-dioxolane sous forme de deux diastéréomères en proportions 62 : 38, est distillé sous pression réduite : sa température d'ébullition est de 49 °C sous 66.7 Pascal.

### Description olfactive : animal, camphré.

Le 2-(3,3-diméthylcyclohex-1-ènyl)-2,4-diméthyl-1,3-dioxolane ainsi obtenu présente les caractéristiques spectrales suivantes :
Isomère majoritaire (62%):
   ¹H-NMR (200 MHz, CDCl₃) : δ (ppm) 0, 93 (s, 6H), 1,25 (d, *J* = 6, 1 Hz, 3H), 1,33-1,38 (m, 2H), 1, 42 (s, 3H), 1, 59-1,61 (m, 2H), 1, 88-1, 95 (m, 2H), 3,37-3,42 (m, 1H), 3,86-3, 90 (m, 1H), 4,02-4,08 (m, 1H), 5, 55 (s, 1H).
   ¹³C-NMR (50 MHz, CDCl₃) : δ (ppm) 18, 99, 19, 87, 24, 00, 24, 71, 29, 92, 29, 96, 31,31, 36, 98, 70, 58, 71,48, 109,56, 132,28, 135,11.
   MS [e/m (%)]: 210 (M+, 0, 3), 195 (100), 137 (24), 109 (34), 101 (998), 93 (10), 91 (14), 81 (11), 79 (15), 76 (15), 67 (16), 43 (64), 41 (16).
Isomère minoritaire (32%) :
   ¹H-NMR (200 MHz, CDCl₃) : δ (ppm) 0, 94 (s, 6H), 1, 22 (d, *J* = 6, 1 Hz, 3H), 1, 33-1, 38 (m, 2H), 1,39 (s, 3H), 1,59-1, 61 (m, 2H), 1, 88-1, 95 (m, 2H), 3, 28-3, 33 (m, 1H), 4,00-4,04 (m, 1H), 4,18-4,25 (m, 1H), 5,60 (s, 1H).
   ¹³C-NMR (50 MHz, CDCl₃) : δ (ppm) 18, 35, 19, 83, 24, 00, 24, 24, 29, 80, 29, 87, 31, 35, 36, 98, 70,92, 72,40, 109,50, 132,54, 136,30.
   MS [e/m (%)]: 210 (M+, 0, 3), 195 (100), 137 (25), 109 (33), 101 (58), 91 (13), 81 (11), 79 (14), 76 (14), 66 (14), 43 (54), 41 (15).
   IR (film, cm⁻¹): 866m, 937w, 953w, 1038s, 1085s, 1187s, 1210s, 1370m, 2930m.

### Exemple 3 : Préparation du 2-(3,3-diméthylcyclohex-1-ènyl)-2,5-diméthyl-1,3-dioxane

Le 2-(3,3-diméthylcyclohex-1-ènyl)-2,5-diméthyl-1,3-dioxane est préparé selon le protocole décrit en exemple 1 en utilisant le 2-méthyl-1,3-propanediol à la place de l'éthylène glycol. Le produit brut constitué de 2-(3,3-diméthylcyclohex-1-ènyl)-2,5-diméthyl-1,3-dioxane, est distillé sous pression réduite : sa température d'ébullition est de 46 °C sous 26.7 Pascal.

### Description olfactive : ambré, vert, fruits rouges.

Le 2-(3,3-diméthylcyclohex-1-ènyl)-2,5-diméthyl-1,3-dioxane ainsi obtenu présente les caractéristiques spectrales suivantes :
¹H-NMR (200 MHz, CDCl₃) : δ (ppm) 0,64 (d, *J* = 6,7 Hz, 3H), 0,85-1,06 (m, 1H), 0,98 (s, 6H), 1,32 (s, 3H), 1, 41-1, 43 (m, 2H), 1, 61-1, 65 (m, 2H), 1, 84 (t, *J* = 6,7 Hz, 1H), 1,97-2,07 (m, 1H), 3,26-3,34 (m, 2H), 3,63-3, 68 (m, 2H), 5, 56 (s, 1H) .
¹³C-NMR (50 MHz, CDCl₃) : δ (ppm) 12, 60, 20, 05, 24,52, 28,69, 28,87, 29,91, 31,82, 37,08, 67,41, 100,23, 132,70, 136,17.
MS [e/m (%)]: 224 (M+, 0, 2), 209 (54), 137 (22), 121 (10), 115 (100), 109 (22), 93 (14), 91 (13), 79 (15), 77 (12), 67 (11), 55 (26), 43 (41), 41 (14).
IR (film, cm⁻¹) : 860m, 1034m, 1052m, 1100m, 1121m, 1162s, 1181s, 1225m, 1368w, 1457w, 2862w, 2930m, 2952m.

### Exemple 4 : Préparation du 2-(3,3-diméthylcyclohex-1-ènyl)-2,5,5-triméthyl-1,3-dioxane

Le 2-(3,3-diméthylcyclohex-1-ènyl)-2,5,5-triméthyl-1,3-dioxane est préparé selon le protocole décrit en exemple 1 en utilisant le néopentyl glycol à la place de l'éthylène glycol. Le produit brut constitué de 2-(3,3-diméthylcyclohex-1-ènyl)-2,5,5-triméthyl-1,3-dioxane, est distillé sous pression réduite : sa température d'ébullition est de 68 °C sous 26.7 Pascal.

### Description olfactive : ambré, musqué, fruits rouges.

Le 2-(3,3-diméthylcyclohex-1-ènyl)-2,5,5-triméthyl-1,3-dioxane ainsi obtenu présente les caractéristiques spectrales suivantes :
¹H-NMR (200 MHz, CDCl₃) : δ (ppm) 0,67 (s, 3H), 0,98 (s, 6H), 1,17 (s, 3H), 1,35 (s, 3H), 1,39-1,43 (m, 2H), 1,61-1,65 (m, 2H), 1,85 (t, *J* = 6,1 Hz, 2H), 3,25-3,28 (m, 2H), 3,42-3,50 (m, 2H), 5,56 (s, 1H).
¹³C-NMR (50 MHz, CDCl₃) : δ (ppm) 20, 05, 22, 15, 22, 70, 24,42, 28, 07, 29,51, 29, 94, 31,79, 37, 09, 71,41, 100,29, 132,63, 135,89.
MS [e/m (%)]: 238 (M+, 0, 4), 223 (45), 137 (20), 129 (100), 109 (24), 93 (15), 91 (14), 81 (12), 79 (14), 77 (12), 69 (28), 67 (14), 55 (13), 43 (57), 41 (24).
IR (film, cm⁻¹) : 859m, 1013m, 1039m, 1083s, 1121m, 1174s, 1207w, 1239w, 1369w, 1470w, 2864w, 2951m.

### Exemple 5 : Préparation du 2-(3,3-diméthylcyclohex-1-ènyl)-5,5-diéthyl-2-méthyl-1,3-dioxane

Le 2-(3,3-diméthylcyclohex-1-ènyl)-5,5-diéthyl-2-méthyl-1,3-dioxane est préparé selon le protocole décrit en exemple 1 en utilisant le 2,2-diéthyl-1,3-propanediol à la place de l'éthylène glycol. Le produit brut constitué de 2-(3,3-diméthylcyclohex-1-ènyl)-5,5-diéthyl-2-méthyl-1,3-dioxane, est distillé sous pression réduite : sa température d'ébullition est de 78 °C sous 40.0 Pascal.

### Description olfactive : faiblement animal.

Le 2-(3,3-diméthylcyclohex-1-ènyl)-5,5-diéthyl-2-méthyl-1,3-dioxane ainsi obtenu présente les caractéristiques spectrales suivantes :
¹H-NMR (200 MHz, CDCl₃) : δ (ppm) 0,72 (t, *J* = 7,5 Hz, 3H), 0,72 (t, *J* = 7,6 Hz, 3H), 0,98 (s, 6H), 0,98-1,05 (m, 1H), 1,26-1,32 (m, 1H), 1,32 (s, 3H), 1,32-1,42 (m, 2H), 1,58-1,73 (m, 4H), 1,82-1,87 (m, 2H), 3,36-3,45 (m, 4H), 5,56 (s, 1H).
¹³C-NMR (50 MHz, CDCl₃) : δ (ppm) 6,61, 7, 50, 20, 05, 22,58, 24,42, 24,48, 27,97, 29,93, 31,76, 34,22, 37,10, 68,38, 100,47, 132,79, 135,79.
MS [e/m (%)]: 208 (M+, 0,1), 134 (19), 133 (46), 132 (17), 121 (11), 119 (13), 110 (11), 109 (10), 108 (13), 107 (17), 97 (17), 96 (10), 95 (13), 93 (23), 92 (11), 91 (34), 81 (26), 80 (10), 79 (35), 77 (14), 67 (28), 57 (100), 55 (23), 53 (11), 41 (49), 39 (12).
IR (film, cm⁻¹) : 838w, 1365m, 1459m, 1726s, 2715w, 2866m, 2928s, 2951s.

### Exemple 6 : Préparation du 2-(3,3-diméthylcyclohex-1-ènyl)-2-méthyl-4,7-dihydro-1,3-dioxepine

Le 2-(3,3-diméthylcyclohex-1-ènyl)-2-méthyl-4,7-dihydro-1,3-dioxepine est préparé selon le protocole décrit en exemple 1 en utilisant le *cis*-2-butène-1,4-diol à la place de l'éthylène glycol. Le produit brut constitué de 2-(3,3-diméthylcyclohex-1-ènyl)-2-méthyl-4,7-dihydro-1,3-dioxepine, est distillé sous pression réduite : sa température d'ébullition est de 75 °C sous 53.3 Pascal.

### Description olfactive : ambré, épicé, muscade, encens.

Le 2-(3,3-diméthylcyclohex-1-ènyl)-2-méthyl-4,7-dihydro-1,3-dioxepine ainsi obtenu présente les caractéristiques spectrales suivantes :
¹H-NMR (200 MHz, CDCl₃) : δ (ppm) 0,98 (s, 6H), 1,37-1,41 (m, 2H), 1,38 (s, 3H), 1,58-1,66 (m, 2H), 1,91-1,96 (m, 2H), 4,10-4,26 (m, 4H), 5,65 (t, *J* = 1,1, 2H), 5,76 (s, 1H).
¹³C-NMR (50 MHz, CDCl₃): δ (ppm) 19,98, 22,41, 24,44, 30,06, 31,55, 37,08, 61,49, 103,40, 129,63, 133,88, 134,33.
MS [e/m (%)]: 222 (M+, 0,1), 207 (15), 154 (33), 152 (22), 137 (58), 109 (62), 93 (14), 91 (14), 81 (18), 79 (16), 77 (14), 70 (10), 69 (13), 67 (27), 55 (14), 53 (13), 43 (100), 42 (19), 41 (38), 39 (29).

IR (film, cm⁻¹) : 613m, 625m, 640m, 787m, 877m, 949m, 1049s, 1082s, 1117m, 1153s, 1200w, 1231m, 1280m, 1371m, 1451w, 2861w, 2930m.

### Exemple 7 : Composition parfumante contenant les dérivés obtenus dans les exemples 1 et 4

A un accord rose réalisé selon le tableau suivant (Accord A) sont ajoutés le 2-(3,3-diméthylcyclohex-1-ènyl)-2,5,5-triméthyl-1,3-dioxane (Exemple 4, Accords B et C) et le 2-(3,3-diméthylcyclohex-1-ènyl)-2-méthyl-1,3-dioxolane (Exemple 1, Accord D).

| Ingrédients | Accord A | Accord B | Accord C | Accord D |
|---|---|---|---|---|
| CITRONELLOL 90% | 100 | 100 | 100 | 100 |
| ALPHA DAMASCONE 10% DPG | 20 | 20 | 20 | 20 |
| DIMETHYLBENZYLCARBINYL ACETATE | 35 | 35 | 35 | 35 |
| EUGENOL RECT. | 5 | 5 | 5 | 5 |
| GERANIOL 95% | 150 | 150 | 150 | 150 |
| GERANYL ACETATE | 50 | 50 | 50 | 50 |
| *CIS*-3-HEXENOL | 30 | 30 | 30 | 30 |
| NEROL 90% | 25 | 25 | 25 | 25 |
| ROSE OXIDE | 10 | 10 | 10 | 10 |
| PHENYLETHYL PHENYLACETATE | 50 | 50 | 50 | 50 |
| PHENYLETHYL ALCOHOL | 400 | 400 | 400 | 400 |
| 2-(3,3-DIMETHYLCYCLOHEX-1-ENYL)-2,5,5-TRIMETHYL-1,3-DIOXANE (Exemple 4) | - | 25 | 50 | - |
| 2-(3,3-DIMETHYLCYCLOHEX-1-ENYL)-2-METHYL-1,3-DIOXOLANE (Exemple 1) | - | - | - | 25 |
| DIPROPYLENE GLYCOL - DPG | 125 | 100 | 75 | 100 |

De manière générale, l'ajout des molécules booste l'accord, le rendant plus puissant.
L'ajout de 2-(3,3-diméthylcyclohex-1-ènyl)-2-méthyl-1,3-dioxolane (Exemple 1, Accord D) donne un effet aromatique, camphré (la rose est moins opulente) tandis que l'ajout du dérivé 2-(3,3-diméthylcyclohex-1-ènyl)-2,5,5-triméthyl-1,3-dioxane (Exemple 4, Accords B et C) renforce la note rose, lui apportant un bel effet naturel...
Au final, les accords B et C sont plus épicés, cinnamiques, doux. L'accord C est cependant plus boisé sec/ambré, la note est moins rosée qu'avec l'accord B (moins dosé), mais l'effet boisé sec s'accorde bien à la composition, donnant de la puissance et une note fleurie boisée, avec un effet baume très agréable.

### Exemple 8 : Composition parfumante contenant les dérivés obtenus dans les exemples 1 et 4

A un accord pomme réalisé selon le tableau suivant (Accord A) sont ajoutés le 2-(3,3-diméthylcyclohex-1-ènyl)-2,5,5-triméthyl-1,3-dioxane (Exemple 4, Accords B et C) et le 2-(3,3-diméthylcyclohex-1-ènyl)-2-méthyl-1,3-dioxolane (Exemple 1, Accord D).

| Ingrédients | Accord A | Accord B | Accord C | Accord D |
|---|---|---|---|---|
| GAMMA UNDECALACTONE | 100 | 100 | 100 | 100 |
| AMYL ISOBUTYRATE | 7 | 7 | 7 | 7 |
| GALAXOLIDE™ 50%MIP | 225 | 225 | 225 | 225 |
| ISOAMYL ACETATE | 22 | 22 | 22 | 22 |
| STYRALLYL ACETATE | 8 | 8 | 8 | 8 |
| TRIPLAL™ | 8 | 8 | 8 | 8 |
| 2-t-BUTYL-CYCLOHEXYL ACETATE 50% DPG | 523 | 523 | 523 | 523 |
| ETHYL METHYLVALERATE | 22 | 22 | 22 | 22 |
| VERTENOL ACETATE | 30 | 30 | 30 | 30 |
| 2-(3,3-DIMETHYLCYCLOHEX-1-ENYL)-2,5,5-TRIMETHYL-1,3-DIOXANE (Exemple 4) | - | 25 | 50 | - |
| 2-(3,3-DIMETHYLCYCLOHEX-1-ENYL)-2-METHYL-1,3-DIOXOLANE (Exemple 1) | - | - | - | 25 |
| DIPROPYLENE GLYCOL - DPG | 55 | 30 | 5 | 30 |

De manière générale, l'ajout des molécules booste l'accord, le rendant plus puissant et plus naturel.
L'ajout de 2-(3,3-diméthylcyclohex-1-ènyl)-2-méthyl-1,3-dioxolane (Exemple 1, Accord D) donne une pomme naturelle, avec un effet peau, acidulé.
L'ajout du dérivé 2-(3,3-diméthylcyclohex-1-ènyl)-2,5,5-triméthyl-1,3-dioxane (Exemple 4, Accords B et C) apporte un effet juteux, sucré, croquant.

### Exemple 9 : Composition parfumante contenant le dérivé obtenu à l'exemple 4

A un accord boisé réalisé selon le tableau suivant (Accord A) est ajouté du 2-(3,3-diméthylcyclohex-1-ènyl)-2,5,5-triméthyl-1,3-dioxane (exemple 4, Accord B) .

| Ingrédients | Accord A | Accord B |
|---|---|---|
| ORCANOX™ | 8 | 8 |
| PENTADECENOLIDE | 125 | 125 |
| HELIOTROPINE CRIST. 10%DPG | 6 | 6 |
| SANDELA™ | 200 | 200 |
| METHYL DIHYDRO JASMONATE | 100 | 100 |
| MANDARINE ESS. DIST. INCOLORE | 50 | 50 |
| IONONE ALPHA | 70 | 70 |
| DEHYDRO 2 CAMPHOLENYL PENTANOL | 4 | 4 |
| DEHYDRO CAMPHOLENYL BUTANOL | 110 | 110 |
| OCTAHYDRO-TETRAMETHYL ACETONAPHTONE | 250 | 250 |
| VANILLINE PARFUMERIE | 10 | 10 |
| DIPROPYLENE GLYCOL - DPG | 67 | 42 |
| 2-(3,3-DIMETHYLCYCLOHEX-1-ENYL)-2,5,5-TRIMETHYL-1,3-DIOXANE (exemple 4) | - | 25 |

L'accord B, poudré, est un peu moins vanillé, plus boisé, masculin.

## Revendications

1. Composé de formule générale I suivante : 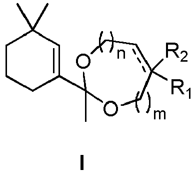 dans laquelle :
- m et n représentent un nombre de carbone et sont chacun indépendamment 0 ou 1;
- R₁ représente un atome d'hydrogène ou un groupe alkyle en C1-C2 saturé;
- la liaison carbone-carbone en pointillé est présente ou absente, et
lorsque ladite liaison est absente, R₂ est présent et représente un atome d'hydrogène ou un groupe alkyle en C1-C2 saturé,
lorsque ladite liaison est présente, R₂ est absent;
ledit composé étant sous forme d'un stéréoisomère, d'un mélange de stéréoisomères, ou d'un mélange racémique.

2. Composé selon la revendication 1, dans lequel ladite liaison carbone-carbone en pointillé est absente.

3. Composé selon la revendication 1, dans lequel n est 0 et m est 1.

4. Composé selon la revendication 1, dans lequel n et m sont 0.

5. Composé selon l'une quelconque des revendications 1 à 4, dans lequel R₁ et R₂ représentent un groupe alkyle en C1-C2 saturé.

6. Composé selon l'une quelconque des revendications précédentes, ledit composé étant choisi parmi le 2-(3,3-diméthylcyclohex-1-ènyl)-2-méthyl-1,3-dioxolane, le 2-(3,3-diméthylcyclohex-1-ènyl)-2,4-diméthyl-1,3-dioxolane, le 2-(3,3-diméthylcyclohex-1-ènyl)-2-méthyl-1,3-dioxane, le 2-(3,3-diméthylcyclohex-1-ènyl)-2,5-diméthyl-1,3-dioxane, le 2-(3,3-diméthylcyclohex-1-ènyl)-2,5,5-triméthyl-1,3-dioxane, le 2-(3,3-diméthylcyclohex-1-ènyl)-5,5-diéthyl-2-méthyl-1,3-dioxane et le 2-(3,3-diméthylcyclohex-1-ènyl)-2-méthyl-4,7-dihydro-1,3-dioxepine.

7. Composition comprenant au moins un composé de formule générale I suivante : 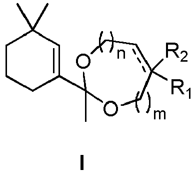 dans laquelle :
- m et n représentent un nombre de carbone et sont chacun indépendamment 0 ou 1;
- R₁ représente un atome d'hydrogène ou un groupe alkyle en C1-C2 saturé;
- la liaison carbone-carbone en pointillé est présente ou absente, et
lorsque ladite liaison est absente, R₂ est présent et représente un atome d'hydrogène ou un groupe alkyle en C1-C2 saturé,
lorsque ladite liaison est présente, R₂ est absent;
ledit composé étant sous forme d'un stéréoisomère, d'un mélange de stéréoisomères, ou d'un mélange racémique.

8. Composition selon la revendication 7, ladite composition comprenant au moins un composé choisi parmi le 2-(3,3-diméthylcyclohex-1-ènyl)-2-méthyl-1,3-dioxolane, le 2-(3,3-diméthylcyclohex-1-ènyl)-2,4-diméthyl-1,3-dioxolane, le 2-(3,3-diméthylcyclohex-1-ènyl)-2-méthyl-1,3-dioxane, le 2-(3,3-diméthylcyclohex-1-ènyl)-2,5-diméthyl-1,3-dioxane, le 2-(3,3-diméthylcyclohex-1-ènyl)-2,5,5-triméthyl-1,3-dioxane, le 2-(3,3-diméthylcyclohex-1-ènyl)-5,5-diéthyl-2-méthyl-1,3-dioxane et le 2-(3,3-diméthylcyclohex-1-ènyl)-2-méthyl-4,7-dihydro-1,3-dioxepine.

9. Composition selon la revendication 7 ou 8, dans laquelle ledit composé est un agent fragrant ; ladite composition comprenant en outre au moins un autre agent fragrant.

10. Composition selon l'une quelconque des revendications 7 à 9, dans laquelle le composé de formule générale I est présent dans une concentration comprise entre 0.01 et 99 % en poids par rapport au poids total de la composition, plus particulièrement entre 0,1 et 30% en poids par rapport au poids total de la composition.

11. Composition selon l'une des revendications 7 à 10, ladite composition étant une composition de parfum.

12. Procédé de préparation d'un composé de formule générale I selon l'une des revendications 1 à 6, ledit procédé comprenant les étapes suivantes :
a) cyclisation / réarrangement du dehydrolinalol par un acide en 1-(3,3-diméthylcyclohex-1-ènyl)éthanone, et ;
b) acétalisation de la 1-(3,3-diméthylcyclohex-1-ènyl)éthanone par un diol pour obtenir le composé de formule générale I.

13. Procédé selon la revendication 12, dans lequel l'acide de l'étape a) est choisi parmi l'acide phosphorique et l'acide méthanesulfonique.

14. Procédé selon l'une des revendications 12 ou 13, dans lequel le diol de l'étape b) est choisi parmi l'éthylène glycol, le néopentyl glycol, le 1,2-propanediol, le 2,2-diéthyl-1,3-propanediol, le 2-méthyl-1,3-propanediol, le 1,3-propanediol, ou encore le *cis*-2-butène-1,4-diol.

15. Utilisation d'au moins un composé de formule générale I selon l'une des revendications 1 à 6 en tant qu'agent fragrant.

16. Utilisation selon la revendication 15, dans laquelle ledit composé de formule générale I est utilisé en combinaison avec au moins un autre agent fragrant et/ou au moins un solvant et/ou au moins un adjuvant.

17. Utilisation selon l'une des revendications 15 ou 16 pour conférer, modifier ou renforcer les propriétés organoleptiques d'une substance, d'une composition ou d'un article.

## Patentansprüche

1. Eine Verbindung der folgenden allgemeinen Formel I: 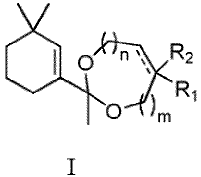 wobei:
- m und n eine Kohlenstoffzahl darstellen und jeweils unabhängig voneinander 0 oder 1 sind;
- R₁ ein Wasserstoffatom oder eine gesättigte C1-C2-Alkylgruppe darstellt;
- die gestrichelte Kohlenstoff-Kohlenstoff-Bindung vorhanden oder nicht vorhanden ist, und
wenn die Bindung nicht vorhanden ist, R₂ vorhanden ist und ein Wasserstoffatom oder eine gesättigte C1-C2-Alkylgruppe darstellt,
wenn die Bindung vorhanden ist, R₂ nicht vorhanden ist;
wobei die Verbindung in Form eines Stereoisomers, einer Mischung aus Stereoisomeren oder einer racemischen Mischung vorliegt.

2. Verbindung gemäß Anspruch 1, wobei die gepunktete Kohlenstoff-Kohlenstoff-Bindung nicht vorhanden ist.

3. Verbindung gemäß Anspruch 1, wobei n 0 ist und m 1 ist.

4. Verbindung gemäß Anspruch 1, wobei n und m 0 sind.

5. Verbindung gemäß einem der Ansprüche 1 bis 4, wobei R₁ und R₂ eine gesättigte C1-C2-Alkylgruppe darstellen.

6. Verbindung gemäß einem der vorhergehenden Ansprüche, wobei die Verbindung ausgewählt ist aus 2-(3,3-Dimethylcyclohex-1-enyl)-2-methyl-1,3-dioxolan, 2-(3,3-Dimethylcyclohex-1-enyl)-2,4-dimethyl-1,3-dioxolan, 2-(3,3-Dimethylcyclohex-1-enyl)-2-methyl-1,3-dioxan, 2-(3,3-Dimethylcyclohex-1-enyl)-2,5-dimethyl-1,3-dioxan, 2-(3,3-Dimethylcyclohex-1-enyl)-2,5,5-trimethyl-1,3-dioxan, 2-(3,3-Dimethylcyclohex-1-enyl)-5,5-diethyl-2-methyl-1,3-dioxan und 2-(3,3-Dimethylcyclohex-1-enyl)-2-methyl-4,7-dihydro-1,3-dioxepin.

7. Eine Zusammensetzung, umfassend mindestens eine Verbindung der folgenden allgemeinen Formel I: 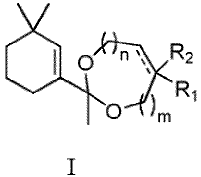 wobei:
- m und n eine Kohlenstoffzahl darstellen und jeweils unabhängig voneinander 0 oder 1 sind;
- R₁ ein Wasserstoffatom oder eine gesättigte C1-C2-Alkylgruppe darstellt;
- die gestrichelte Kohlenstoff-Kohlenstoff-Bindung vorhanden oder nicht vorhanden ist, und
wenn die Bindung nicht vorhanden ist, R₂ vorhanden ist und ein Wasserstoffatom oder eine gesättigte C1-C2-Alkylgruppe darstellt,
wenn die Bindung vorhanden ist, R₂ nicht vorhanden ist;
wobei die Verbindung in Form eines Stereoisomers, einer Mischung aus Stereoisomeren oder einer racemischen Mischung vorliegt.

8. Zusammensetzung gemäß Anspruch 7, wobei die Zusammensetzung mindestens eine Verbindung umfasst, ausgewählt aus 2-(3,3-Dimethylcyclohex-1-enyl)-2-methyl-1,3-dioxolan, 2-(3,3-Dimethylcyclohex-1-enyl)-2,4-dimethyl-1,3-dioxolan, 2-(3,3-Dimethylcyclohex-1-enyl)-2-methyl-1,3-dioxan, 2-(3,3-Dimethylcyclohex-1-enyl)-2,5-dimethyl-1,3-dioxan, 2-(3,3-Dimethylcyclohex-1-enyl)-2,5,5-trimethyl-1,3-dioxan, 2-(3,3-Dimethylcyclohex-1-enyl)-5,5-diethyl-2-methyl-1,3-dioxan und 2-(3,3-Dimethylcyclohex-1-enyl)-2-methyl-4,7-dihydro-1,3-dioxepin.

9. Zusammensetzung gemäß Anspruch 7 oder 8, wobei die Verbindung ein Duftstoff ist; wobei die Zusammensetzung ferner mindestens einen weiteren Duftstoff umfasst.

10. Zusammensetzung gemäß einem der Ansprüche 7 bis 9, wobei die Verbindung der allgemeinen Formel I in einer Konzentration von zwischen 0,01 und 99 Gew.-%, bezogen auf das Gesamtgewicht der Zusammensetzung, insbesondere von zwischen 0,1 und 30 Gew.-%, bezogen auf das Gesamtgewicht der Zusammensetzung, vorhanden ist.

11. Zusammensetzung gemäß einem der Ansprüche 7 bis 10, wobei die Zusammensetzung eine Parfümzusammensetzung ist.

12. Ein Verfahren zur Herstellung einer Verbindung der allgemeinen Formel I gemäß einem der Ansprüche 1 bis 6, wobei das Verfahren die folgenden Schritte umfasst:
a) Cyclisieren/Umlagern von Dehydrolinalol mit einer 1-(3,3-Dimethylcyclohex-1-enyl)ethanonsäure, und;
b) Acetalisieren von 1-(3,3-Dimethylcyclohex-1-enyl)ethanon mit einem Diol, um die Verbindung der allgemeinen Formel I zu erhalten.

13. Verfahren gemäß Anspruch 12, wobei die Säure von Schritt a) aus Phosphorsäure und Methansulfonsäure ausgewählt ist.

14. Verfahren gemäß einem der Ansprüche 12 oder 13, wobei das Diol von Schritt b) aus Ethylenglykol, Neopentylglykol, 1,2-Propandiol, 2,2-Diethyl-1,3-propandiol, 2-Methyl-1,3-propandiol, 1,3-Propandiol oder *cis*-2-Buten-1,4-diol ausgewählt ist.

15. Eine Verwendung mindestens einer Verbindung der allgemeinen Formel I gemäß einem der Ansprüche 1 bis 6 als Duftstoff.

16. Verwendung gemäß Anspruch 15, wobei die Verbindung der allgemeinen Formel I in Kombination mit mindestens einem anderen Duftstoff und/oder mindestens einem Lösungsmittel und/oder mindestens einem Adjuvans verwendet wird.

17. Verwendung gemäß einem der Ansprüche 15 oder 16, um die organoleptischen Eigenschaften einer Substanz, einer Zusammensetzung oder eines Artikels zu verleihen, zu modifizieren oder zu verstärken.

## Claims

1. Compound of the following general formula I: 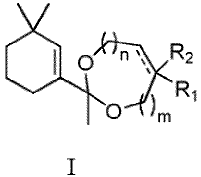 wherein:
- m and n represent a carbon number and are each independently 0 or 1;
- R₁ represents a hydrogen atom or a saturated C1-C2 alkyl group;
- the carbon-carbon bond shown as a dotted line is present or absent, and
when said bond is absent, R₂ is present and represents a hydrogen atom or a saturated C1-C2 alkyl group,
when said bond is present, R₂ is absent;
said compound being in the form of a stereoisomer, a mixture of stereoisomers, or a racemic mixture.

2. Compound according to claim 1, wherein said carbon-carbon bond shown as a dotted line is absent.

3. Compound according to claim 1, wherein n is 0 and m is 1.

4. Compound according to claim 1, wherein n and m are 0.

5. Compound according to any one of claims 1 to 4, wherein R₁ and R₂ represent a saturated C1-C2 alkyl group.

6. Compound according to any one of the previous claims, said compound being chosen from 2-(3,3-dimethylcyclohex-1-enyl)-2-methyl-1,3-dioxolane, 2-(3,3-dimethylcyclohex-1-enyl)-2,4-dimethyl-1,3-dioxolane, 2-(3,3-dimethylcyclohex-1-enyl)-2-methyl-1,3-dioxane, 2-(3,3-dimethylcyclohex-1-enyl)-2,5-dimethyl-1,3-dioxane, 2-(3,3-dimethylcyclohex-1-enyl)-2,5,5-trimethyl-1,3-dioxane, 2-(3,3-dimethylcyclohex-1-enyl)-5,5-diethyl-2-methyl-1,3-dioxane and 2-(3,3-dimethylcyclohex-1-enyl)-2-methyl-4,7-dihydro-1,3-dioxepine.

7. Composition comprising at least one compound of the following general formula I: 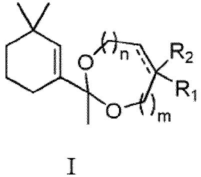 wherein:
- m and n represent a carbon number and are each independently 0 or 1;
- R₁ represents a hydrogen atom or a saturated C1-C2 alkyl group;
- the carbon-carbon bond shown as a dotted line is present or absent, and
when said bond is absent, R₂ is present and represents a hydrogen atom or a saturated C1-C2 alkyl group,
when said bond is present, R₂ is absent;
said compound being in the form of a stereoisomer, a mixture of stereoisomers, or a racemic mixture.

8. Composition according to claim 7, said composition comprising at least one compound chosen from 2-(3,3-dimethylcyclohex-1-enyl)-2-methyl-1,3-dioxolane, 2-(3,3-dimethylcyclohex-1-enyl)-2,4-dimethyl-1,3-dioxolane, 2-(3,3-dimethylcyclohex-1-enyl)-2-methyl-1,3-dioxane, 2-(3,3-dimethylcyclohex-1-enyl)-2,5-dimethyl-1,3-dioxane, 2-(3,3-dimethylcyclohex-1-enyl)-2,5,5-trimethyl-1,3-dioxane, 2-(3,3-dimethylcyclohex-1-enyl)-5,5-diethyl-2-methyl-1,3-dioxane and 2-(3,3-dimethylcyclohex-1-enyl)-2-methyl-4,7-dihydro-1,3-dioxepine.

9. Composition according to claim 7 or 8, wherein said compound is a fragrant agent; said composition further comprising at least one other fragrant agent.

10. Composition according to any one of claims 7 to 9, wherein the compound of general formula I is present in a concentration of between 0.01 and 99 wt% of the total weight of the composition, more particularly between 0.1 and 30 wt% of the total weight of the composition.

11. Composition according to one of claims 7 to 10, said composition being a perfume composition.

12. Method of preparing a compound of general formula I according to one of claims 1 to 6, said method comprising the following steps:
a) cyclisation/rearrangement of dehydrolinalool by an acid into 1-(3,3-dimethylcyclohex-1-enyl)ethanone, and;
b) acetalisation of 1-(3,3-dimethylcyclohex-1-enyl)ethanone by a diol to obtain the compound of general formula I.

13. Method according to claim 12, wherein the acid in step a) is chosen from phosphoric acid and methanesulfonic acid.

14. Method according to one of claims 12 or 13, wherein the diol in step b) is chosen from ethylene glycol, neopentyl glycol, 1,2-propanediol, 2,2-diethyl-1,3-propanediol, 2-methyl-1,3-propanediol, 1,3-propanediol, or *cis*-2-butene-1,4-diol.

15. Use of at least one compound of general formula I according to one of claims 1 to 6 as a fragrant agent.

16. Use according to claim 15, wherein said compound of general formula I is used in combination with at least one other fragrant agent and/or at least one solvent and/or at least one additive.

17. Use according to one of claims 15 or 16 to confer, alter or reinforce the organoleptic properties of a substance, composition or item.
